(19) Europäisches Patentamt
European Patent Office
Office européen des brevets

(11) Veröffentlichungsnummer: **0 175 187**
**B1**

(12) # EUROPÄISCHE PATENTSCHRIFT

(45) Veröffentlichungstag der Patentschrift: **10.01.90**

(51) Int. Cl.⁴: **C 07 D 401/10**, C 07 D 409/14, A 61 K 31/44

(21) Anmeldenummer: **85110906.6**

(22) Anmeldetag: **29.08.85**

(54) **Neue, eine Imidazol-gruppe enthaltende 3,4-Dihydro-2(1H)-Pyridone und 2(1H)-Pyridone, Verfahren zu ihrer Herstellung und diese enthaltende Arzneimittel.**

(30) Priorität: **15.09.84 DE 3433953**

(43) Veröffentlichungstag der Anmeldung: **26.03.86 Patentblatt 86/13**

(45) Bekanntmachung des Hinweises auf die Patenterteilung: **10.01.90 Patentblatt 90/2**

(84) Benannte Vertragsstaaten: **AT BE CH DE FR GB IT LI LU NL SE**

(56) Entgegenhaltungen:
**EP-A- 0 102 227**
**AT-B- 323 186**
**DE-A- 3 016 874**

(73) Patentinhaber: **A. Nattermann & Cie. GmbH, Nattermannallee 1, D-5000 Köln 30 (DE)**

(72) Erfinder: **Hillboll, Gerd, Dr., Neusser Gasse 30, D-5024 Pulheim (DE)**
Erfinder: **Doppelfeld, Ilse-Stephanie, Im Bruchfeldchen 35, D-5010 Bergheim-Glessen (DE)**
Erfinder: **Prop, Gerrit, Dr., Anemonenweg 23, D-5024 Pulheim (DE)**

(74) Vertreter: **Redies, Bernd, Dr. rer. nat. et al, COHAUSZ & FLORACK Patentanwaltsbüro Schumannstrasse 97 Postfach 14 01 20, D-4000 Düsseldorf 1 (DE)**

**EP 0 175 187 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue 3,4-Dihydro-2(1H)-pyridone und 2(1H)-Pyridone, die in 5-Stellung durch eine Imidazolylthienyl-Gruppe substituiert sind, sowie deren Herstellung und deren Verwendung als Arzneimittel zur Behandlung von Herzinsuffizienz.

Die Europäische Patentanmeldung 109 628 beschreibt 3,4-Dihydro-2(1H)-pyridone und 2(1H)-Pyridone, die in 5-Stellung durch Hydroxyphenyl-, Methoxyphenyl- oder Pyridyl-Gruppen substituiert sind sowie ihre potentielle Anwendung als Cardiotonika.

In der Europäischen Patentanmeldung 102 227 werden unter anderem positiv inotrop wirkende 5-(Imidazolylphenyl)-2(1H)-pyridone beschrieben, die in 3-Stellung durch H, $CH_2OH$, F, Cl, Br, CN, COOR, CNR′R″, NR′R″ substituiert sein können.

Die vorliegende Erfindung beinhaltet 3,4-Dihydro-2(1H)-pyridone der Formel I,

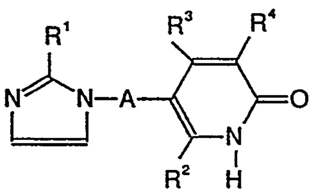 I

in der
===== eine Einfachbindung zwischen zwei Kohlenstoffatomen bedeutet,
$R^1$, $R^3$, $R^4$ unabhängig voneinander Wasserstoff oder einen Methylrest,
$R^2$ einen $C_{1-4}$-Niederalkylrest bedeuten und

A für 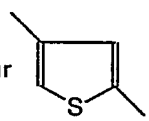 oder 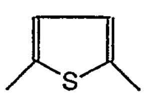 steht.

Ebenfalls Gegenstand der vorliegenden Erfindung und bevorzugt sind 2(1H)-Pyridone der Formel I, in der
===== eine Doppelbindung zwischen zwei Kohlenstoffatomen bedeutet,

A für 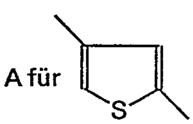 oder 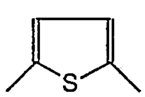 steht,

$R^1$, $R^3$ unabhängig voneinander Wasserstoff oder einen Methylrest,
$R^2$ einen $C_{1-4}$-Niederalkylrest und
$R^4$ Wasserstoff, Methyl, COOH, CN, $CONH_2$ oder $NH_2$ bedeuten.

Die 3,4-Dihydro-2(1H)-pyridone der Formel I (===== bedeutet Einfachbindung) besitzen ein Chiralitätszentrum an den Positionen 3 bzw. 4 des Pyridinringes, wenn die Substituenten $R^3$ und/oder $R^4$ ungleich Wasserstoff sind und können somit als Racemate oder in Form der Enantiomeren vorliegen. Falls eine Trennung der Racemate erwünscht ist, wird diese nach an sich bekannten Verfahren mit einer optisch aktiven Säure, wie z.B. Dibenzoylweinsäure oder Campher-10-sulfonsäure, über die Bildung diastereomerer Salze oder durch Chromatographie an optisch aktivem Säulenmaterial durchgeführt.

Die Erfindung umfasst auch die pharmazeutisch verwendbaren Säureadditionssalze von Verbindungen der Formal I, z.B. solche mit Chlorwasserstoffsäure, Bromwasserstoffsäure, Schwefelsäure oder Phosphorsäure oder mit organischen Säuren, z.B. Essigsäure, Propionsäure, Oxalsäure, Malonsäure, Glykolsäure, Bernsteinsäure, Maleinsäure, Fumarsäure, Äpfelsäure, Zitronensäure, Weinsäure, Milchsäure, Benzoesäure.

Verbindungen gemäss der vorliegenden Erfindung sind beispielsweise:

3,4-Dihydro-5-[5-(1-imidazolyl)-2-thienyl]-6-methyl-2(1H)-pyridon
3,4-Dihydro-5-[4-(1-imidazolyl)-2-thienyl]-6-methyl-2(1H)-pyridon
3,4-Dihydro-3,6-dimethyl-5-[4-(1-imidazolyl)-2-thienyl]-2(1H)-pyridon
3,4-Dihydro-3,6-dimethyl-5-[5-(1-imidazolyl)-2-thienyl]-2(1H)-pyridon
3,4-Dihydro-4,6-dimethyl-5-[5-(1-imidazolyl)-2-thienyl]-2(1H)-pyridon
3,4-Dihydro-4,6-dimethyl-5-[4-(1-imidazolyl)-2-thienyl]-2(1H)-pyridon
3,4-Dihydro-6-ethyl-5-[5-(1-imidazolyl)-2-thienyl]-2(1H)-pyridon
3,4-Dihydro-6-ethyl-5-[5-(1-imidazolyl)-2-thienyl]-3-methyl-2(1H)-pyridon
3,4-Dihydro-6-ethyl-5-[4-(1-imidazolyl)-2-thienyl)-2(1H)-pyridon
3,4-Dihydro-6-ethyl-5-[4-(1-imidazolyl)-2-thienyl]-3-methyl-2(1H)-pyridon
5-[5-(1-Imidazolyl)-2-thienyl]-6-methyl-2(1H)-pyridon
5-[4-(1-Imidazoyl)-2-thienyl]-6-methyl-2(1H)-pyridon
3,6-Dimethyl-5-[5-(1-imidazolyl)-2-thienyl]-2(1H)-pyridon
3,6-Dimethyl-5-[4-(1-imidazolyl)-2-thienyl]-2(1H)-pyridon
4,6-Dimethyl-5-[5-(1-imidazolyl)-2-thienyl]-2(1H)-pyridon
4,6-Dimethyl-5-[4-(1-imidazolyl)-2-thienyl]-2(1H)-pyridon
6-Ethyl-5-[5-(1-imidazolyl)-2-thienyl]-2(1H)-pyridon
6-Butyl-5-[4-(1-imidazolyl)-2-thienyl]-3-methyl-2(1H)-pyridon
6-Butyl-5-[5-(1-imidazolyl)-2-thienyl]-3-methyl-2(1H)-pyridon
5-[4-(1-imidazolyl)-2-thienyl]-3-methyl-6-propyl-2(1H)-pyridon
5-[5-(1-imidazolyl)-2-thienyl]-3-methyl-6-propyl-2(1H)-pyridon
6-Ethyl-5-[5-(1-imidazolyl)-2-thienyl]-3-methyl-2(1H)-pyridon

6-Ethyl-5-[4-(1-imidazolyl)-2-thienyl]-2(1H)-pyridon
6-Ethyl-5-[4-(1-imidazolyl)-2-thienyl]-3-methyl-2(1H)-pyridon
1,2-Dihydro-5-[5-(1-imidazolyl)-2-thienyl]-6-methyl-2-oxopyridin-3-carbonsäurenitril
1,2-Dihydro-5-[4-(1-imidazolyl)-2-thienyl]-6-methyl-2-oxopyridin-3-carbonsäurenitril
1,2-Dihydro-4,6-dimethyl-5-[4-(1-imidazolyl)-2-thienyl]-2-oxo-pyridin-3-carbonsäurenitril
1,2-Dihydro-5-[5-(1-imidazolyl)-2-thienyl]-6-methyl-2-oxopyridin-3-carbonsäure
1,2-Dihydro-5-[4-(1-imidazolyl)-2-thienyl]-6-methyl-2-oxopyridin-3-carbonsäureamid
1,2-Dihydro-5-[5-(1-imidazolyl)-2-thienyl]-6-methyl-2-oxopyridin-3-carbonsäureamid
1,2-Dihydro-5-[4-(1-imidazolyl)-2-thienyl]-6-methyl-2-oxopyridin-3-carbonsäureamid
3-Amino-5-[5-(1-imidazolyl)-2-thienyl]-6-methyl-2(1H)-pyridon
3-Amion-5-[4-(1-imidazolyl)-2-thienyl]-6-methyl-2(1H)-pyridon

Die Herstellung der erfindungsgemässen Verbindungen erfolgt nach oder analog bekannten Verfahren. So werden die 3,4-Dihydro-2(1H)-pyridon der Formel I, in der ═══ eine Einfachbindung zwischen zwei Kohlenstoffatomen darstellt, $R^1$, $R^3$ und $R^4$ unabhängig voneinander Wasserstoff oder einen Methylrest und $R^2$ eine $C_{1-4}$-Niederalkylgruppe bedeuten sowie A für

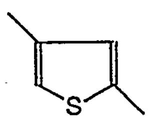 oder 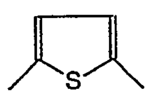 steht,

durch Umsetzung eines Alkanon der Formel IV

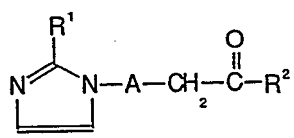 IV

in der A Phenylen oder Thienylen bedeutet und $R^2$ einen $C_{1-4}$-Niederalkylrest darstellt, mit einem Acrylsäureamid der Formel V

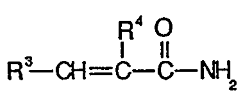 V

in der $R^3$ und $R^4$ Wasserstoff oder eine Methylgruppe bedeuten, hergestellt.

Die Reaktion wird in einem inerten Lösungsmittel, wie z.B. 1,4-Dioxan, Dimethylformamid, Tetrahydrofuran, N-Methyl-pyrrolidon, Dimethylsulfoxid, Sulfolan, Methanol, Ethanol in Gegenwart einer starken Base, wie z.B. Kalium-tert-butylat, Natriumhydrid, Natriummethylat, Natriumethylat, Natriumamid, bevorzugt in 1,4-Dioxan mit Kalium-tert-butylat als Base bei Raumtemperatur durchgeführt. Wenn nötig, können die Reaktionsansätze zur Kondensation der intermediär durch Michael-Addition entstehenden und auch isolierbaren 5-Oxo-pentansäureamide der Formel VIII,

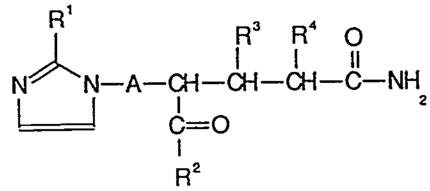 VIII

in der $R^1$, $R^2$, $R^3$, $R^4$ und A die oben angegebenen Bedeutungen haben, kurzzeitig auf Temperaturen bis etwa 100°C erhitzt werden.

Die als Ausgangsverbindungen der Formel IV eingesetzten Alkanone können z.B. nach oder analog EP 102 227 hergestellt werden.

Als Ausgangsverbindungen der Formel IV kommen z.B. in Frage:

1-[5-(1-Imidazolyl)-2-thienyl]-2-propanon
1-[5-(1-Imidazolyl)-2-thienyl]-2-butanon
1-[5-(1-Imidazolyl)-2-thienyl)-2-pentanon
1-[5-(1-Imidazolyl)-2-thienyl)-2-hexanon
1-[4-(1-Imidazolyl)-2-thienyl)-2-propanon
1-[4-(1-Imidazolyl)-2-thienyl)-2-butanon
1-[4-(1-Imidazolyl)-2-thienyl)-2-pentanon
1-[4-(1-Imidazolyl)-2-thienyl)-2-hexanon
1-[5-(2-Methyl-1-imidazolyl)-2-thienyl]-2-propanon

Als Acrylsäureamide der Formel V können z.B. eingesetzt werden: Acrylsäureamid, Crotonsäureamid, Methacrylsäureamid, Tiglinsäureamid.

Alternativ können die 3,4-Dihydro-2(1H)-pyridon der Formel I hergestellt werden durch Michael-Reaktion der Alkanone der Formel IV mit den Acrylsäurenitrilen der Formel IX,

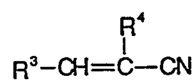 IX

in der $R^3$ und $R^4$ für Wasserstoff oder eine Methylgruppe stehen, und anschliessende Behandlung der entstandenen 5-Oxo-pentansäurenitrile der Formel II,

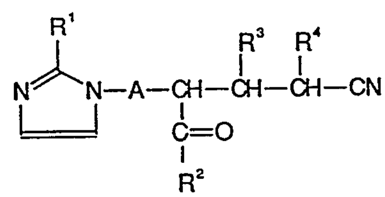 II

in der A, $R^1$, $R^2$, $R^3$, $R^4$ die oben angegebenen Bedeutungen haben, mit starken Säuren. Dabei wird die Umsetzung der Alkanonen mit den Acrylsäurenitrilen bei Temperaturen zwischen 0 und 100°C in Gegenwart einer starken Base, wie z.B. Natriumhydrid, Natriumethylat, Natriummethylat, Kalium-tert-butylat, Natriumamid, bevorzugt Natriummethylat, in einem organischen Lösungsmittel, wie z..B. Methanol, Ethanol, 1,4-Dioxan, Tetrahydrofuran, Dimethylformamid, N-Methylpyrrolidon, Dimethylsulfoxid, bevorzugt in Methanol durchgeführt. Die Umwandlung der 5-Oxo-pentansäurenitrile der Formel II in die 3,4-Dihydro-2(1H)-pyridone der Formel I wird durch Behandlung mit starken Mineralsäuren,

wie z. B. konzentrierter Schwefelsäure, Salzsäure, Polyphosphorsäure, gegebenenfalls in einem geeigneten Lösungsmittel, wie z. B. Essigsäure, Ethanol, bevorzugt durch Behandlung mit Schwefelsäure in Essigsäure bei Temperaturen zwischen 0 und ca. 100°C erreicht. Als Verbindungen der Formel IX kommen in Frage: Acrylsäurenitril, Crotonsäurenitril, Methacrylsäurenitril, Tiglinsäurenitril.

Eine dritte Möglichkeit zur Herstellung der 3,4-Dihydro-2(1H)-pyridone der Formel I besteht in der Umsetzung der Alkanone der Formel IV mit Acrylsäureestern der Formel X,

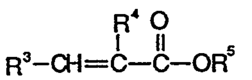
X

in der $R^3$, $R^4$ Wasserstoff oder eine Methylgruppe bedeuten und $R^5$ ein $C_{1-4}$-Niederalkylrest ist, und anschliessende Umsetzung der erhaltenen 5-Oxo-pentansäureester der Formel III,

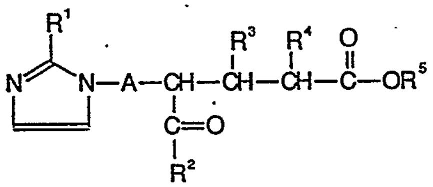
III

in der A, $R^1$, $R^2$, $R^3$, $R^4$, $R^5$ die oben angegebenen Bedeutungen haben, mit Ammoniak oder einem geeigneten Ammoniumsalz. Dabei wird die Umsetzung der Alkanone der Formel IV mit den Acrylsäureestern der Formel X bei Temperaturen zwischen 0 und 100°C, gegebenenfalls in einem geeigneten organischen Lösungsmittel, wie z. B. Methanol, Ethanol, Dimethylformamid, Dimethylsulfoxid in Anwesenheit von Benzyltrimethylammoniumhydrochlorid oder eines anderen geeigneten Katalysators durchgeführt. Die weitere Umsetzung der Verbindungen der Formel III mit Ammoniak oder einem geeigneten Ammoniumsalz, wie z. B. Ammoniumacetat, Ammoniumchlorid, Ammoniumcarbonat, Ammoniumformiat, wird bei Temperaturen von 0–100°C in einem geeigneten Lösungsmittel, wie Ethanol, Methanol, 1,4-Dioxan, Dimethylformamid, Dimethylsulfoxid, vorgenommen, wobei bevorzugt mit Ammoniak unter Druck gearbeitet wird.

Als Ausgangsverbindungen der Formel X können z. B. eingesetzt werden:

Acrylsäuremethylester, Acrylsäureethylester, Acrylsäurepropylester, Acrylsäurebutylester, Crotonsäuremethylester, Crotonsäureethylester, Methacrylsäuremethylester, Methacrylsäureethylester, Tiglinsäuremethylester, Tiglinsäureethylester.

Die Herstellungsverfahren für die 3,4-Dihydro-2(1H)-pyridone der Formel I werden durch folgendes Formelschema verdeutlicht:

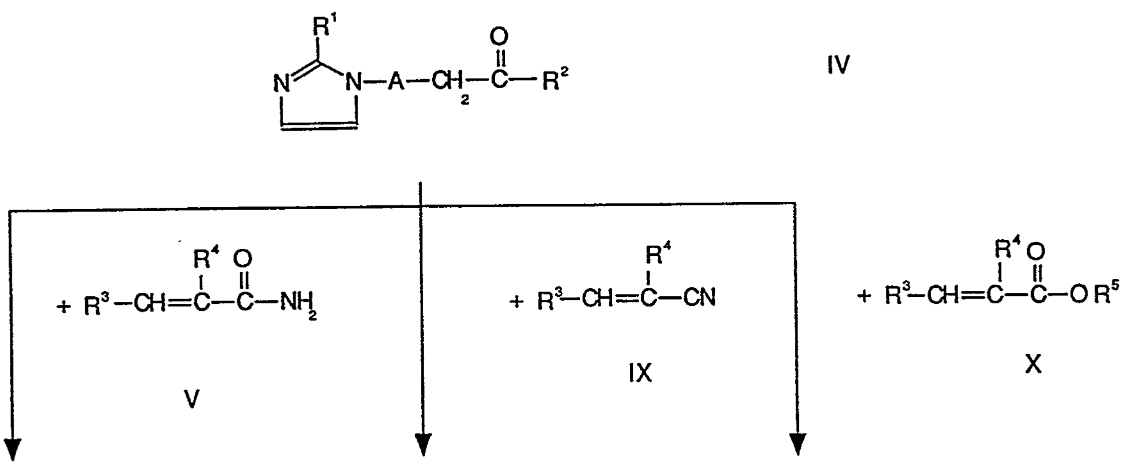
IV

V

IX

X

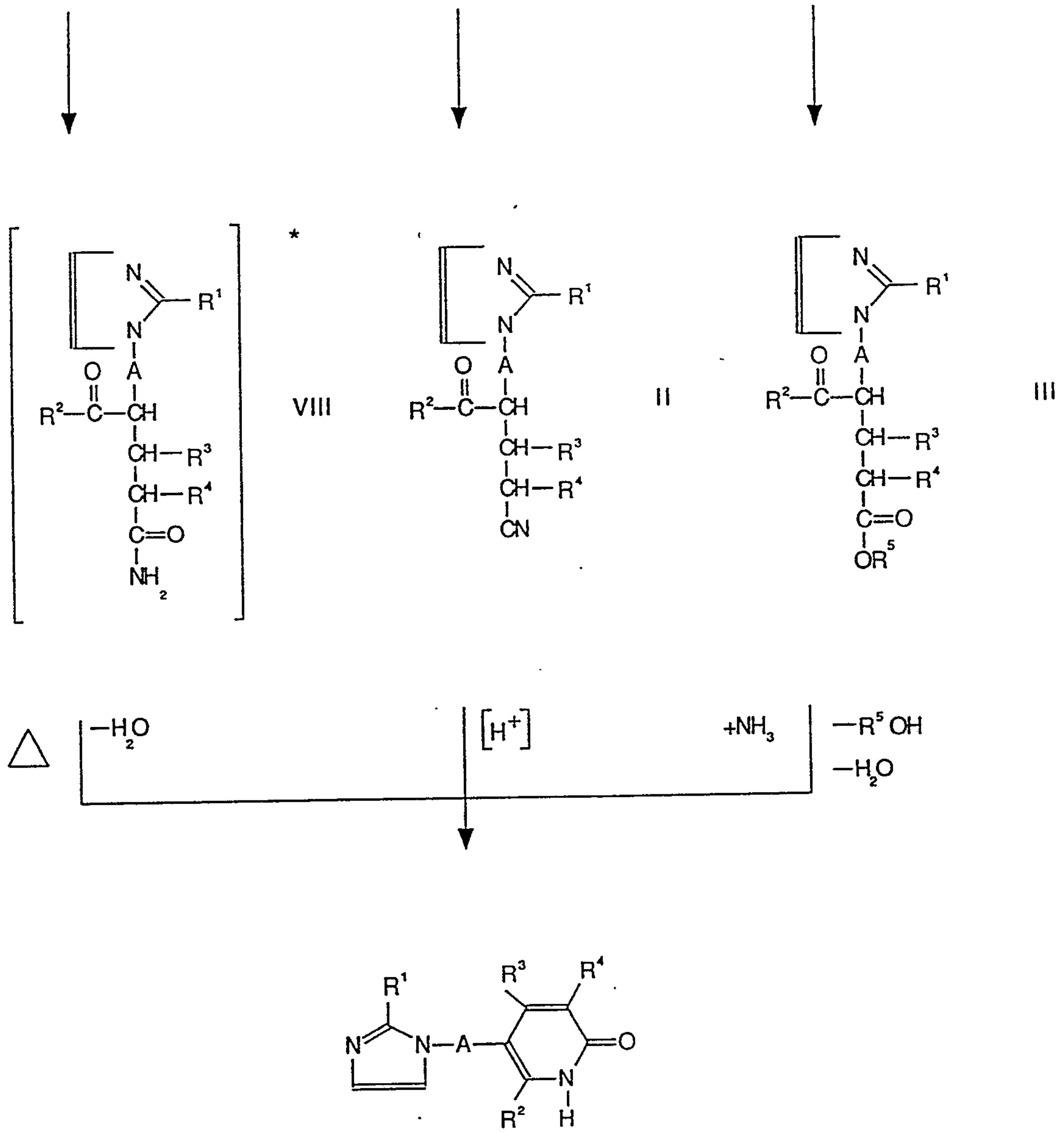


(------ bedeutet Einfachbindung)

* isolierbare Zwischenstufe

Die Herstellung der 2(1H)-Pyridone der Formel I (------ bedeutet Doppelbindung) mit $R^4$ = Wasserstoff, Methyl erfolgt analog bekannten Verfahren durch Umwandlung der entsprechenden 3,4-Dihydro-2(1H)-pyridone der Formel I.

Diese Umwandlung kann durch Dehydrierung der 3,4-Dihydro-2(1H)-pyridone der Formel I in Gegenwart eines Edelmetallkatalysators mit oder ohne Träger, wie z.B. Palladium, in einem hochsiedenden organischen Lösungsmittel, wie z.B. Diethylenglykoldimethylether, Diethylenglykolmethyl-tert-butylether, Diphenylether, bei Rückflusstemperatur erfolgen.

Eine zweite Umwandlungsmöglichkeit besteht im Erhitzen von 3,4-Dihydro-2(1H)-pyridonen der Formel I mit Schwefel in einem hochsiedenden organischen Lösungsmittel, wie z.B. Diethylphthalat oder einer Mischung aus Diphenyl und Diphenylether auf Temperaturen von 150–250°C.

Die oben genannte Umwandlung kann auch durch Behandlung der 3,4-Dihydro-2(1H)-pyridone der Formel I mit Brom in Essigsäure bei Temperaturen zwischen 20°C und Rückflusstemperatur durchgeführt werden. Bevorzugt werden die 3,4-Dihydro-2(1H)-pyridone der Formel I durch Reaktion mit 3-Nitrobenzolsulfonsäure in alkalisch-wässrigem Milieu bei Temperaturen von 60–100°C in die entsprechenden 2(1H)-Pyridone der Formel I überführt.

Die 2(1H)-Pyridone der Formel I (------ bedeutet Doppelbindung) mit $R^3 = CH_3$, $R^4 = H$, A = Thienylen können auch hergestellt werden durch Reaktion der Alkanone der Formel IV (A = Thienylen) mit Acetoacetamid in einer Mischung aus Polyphosphorsäure und Methansulfonsäure bei Temperaturen von 70 °C bis 150 °C, bevorzugt bei 110 °C bis 130 °C.

Die 2(1H)-Pyridone der Formel I ( ====== bedeutet Doppelbindung) mit $R^4$ = CN werden analog zu bekannten Verfahren hergestellt.

Ausgehend von den Alkanonen der Formel IV erhält man durch Umsetzung mit N,N-Dimethylformamid-dimethylacetal bzw. N,N-Dimethylacetamid-dimethylacetal der Formel VI,

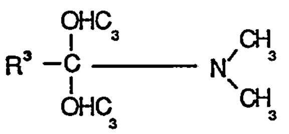

VI

in der $R^3$ Wasserstoff oder eine Methylgruppe ist, in einem geeigneten Lösungsmittel, wie z. B. Acetonitril, die 2-(Dimethylamino)-ethenylalkanone der Formel VII,

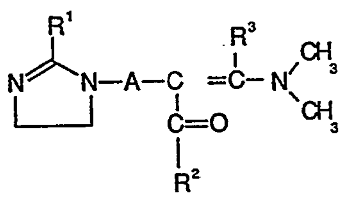

VII

in der $R^1$, $R^2$, $R^3$ und A die oben angegebenen Bedeutungen haben. Diese werden in einem geeigneten Lösungsmittel, wie z. B. N,N-Dimethylformamid, in Gegenwart einer starken Base, wie z. B. Natriumhydrid, Natriumethylat, bei Temperaturen von 50–150 °C mit Cyanacetamid zu den 2(1H)-Pyridonen der Formel I mit $R^4$ = CN umgesetzt.

Diese Nitrile der Formel I können nach bekannten Verfahren weiter umgewandelt werden, so z. B. durch Hydrolyse in starken Mineralsäuren, wie konzentrierter Schwefelsäure, konzentrierter Salzsäure, in die entsprechenden Carbonsäureamide der Formel I oder Carbonsäuren der Formel I, die wiederum thermisch durch Decarboxylierung in einem geeigneten Lösungsmittel, wie z. B. Diphenylether, bei Temperaturen bis 250 °C in die entsprechenden 2(1H)-Pyridone der Formel I ( ====== bedeutet Doppelbindung) mit $R^4$ = Wasserstoff überführt werden können. Ebenso können die Carbonsäureamide bzw. die Carbonsäuren der Formel I durch bekannte Abbauverfahren, wie z. B. Hoffmann-Abbau, Lossen-Abbau, Curtius-Abbau, in die entsprechenden Amine der Formel I ( ====== bedeutet Doppelbindung, $R^4$ = $NH_2$) überführt werden.

Die vorteilafte Verwendung der Verbindungen der Formel I als Cardiotonica wird durch ihre Wirkung in pharmakologischen Standard-Testen demonstriert, wie z. B. in der Erhöhung der Kontraktilität von isolierten Meerschweinchen-Atrien und/oder in der Erhöhung der Herz-Kontraktilität von anästhesierten Katzen oder Hunden bei gleichzeitig nur geringen Änderungen von Herzfrequenz und Blutdruck.

Neben der positiv inotropen Wirkung zeigen die erfindungsgemässen Verbindungen eine starke Hemmung der durch Collagen induzierten Aggregation menschlicher Thrombozyten in vitro.

Auswertung der in vivo cardiotonischen Aktivität

Anästhetisierter Hund

Die Wirkungen ansteigender Dosen der Wirkstoffe wurde nach intravenöser Gabe an anästhetisierten Hunden gemessen. Als Parameter wurden myocardiale Kontraktilität, Herzschlagzahl und arterieller Blutdruck aufgezeichnet.

Verfahren

Erwachsene Mongrel-Hunde beider Geschlechter erhielten eine Vormedikation von Morphinhydrochlorid (1,0 mg/kg s.c.). 30 Minuten später wurde die Anästhesie mittels Chloralose/Harnstoff eingeleitet. Die Tiere wurden mit 500 I.E. Heparin/kg (i.v.) als Anfangsdosis heparinisiert, gefolgt von alle 4 Stunden gegebenen Dosen von 250 I.E./kg.

Die Luftröhre wurde intubiert, jedoch liess man zu, dass die Tiere spontan atmeten. Die Testlösungen wurden mittels einer Injektionsnadel in die rechtsseitige Vena cephalica antebrachii injiziert. Arterieller Blutdruck wurde mittels einer Injektionsnadel in der Arteria femoralis gemessen. Um die myocardische Kontraktilität aufzeichnen zu können, wurde ein Millar-Katheter-Spitzendrucküberträger in die linke Ventrikel über die linke Carotisarterie eingeführt. Der arterielle Blutdruck und der Blutdruck in der linken Ventrikel wurden laufend gemessen. Die Herzschlagrate ergab sich aus den Spitzen der Blutdrucksignale.

Aus dem linksseitigen Ventrikelblutdruck wurde die erste Ableitung mittels eines Differentialamplifikators berechnet. Dies ergab den linksseitigen Ventrikelwert dp/dt, der auch aufgezeichnet wurde. Die getesteten Verbindungen wurden in Tetrahydrofurfurol-polyethylenglycolether gelöst, falls notwendig, unter Erhitzen.

Jede Dosis der untersuchten Verbindungen wurde in einem Volumen von 0,1 ml/kg verabreicht, wobei die Dauer der Injektion 1 Minute betrug.

Die Ergebnisse mit der erfindungsgemässen Testsubstanz 3,6-Dimethyl-5-[4-(1-imidazolyl)-2-thienyl]-2-(1H)-pyridon-hydrochlorid = A im Vergleich zur vorbekannten Verbindung 4,5-Dihydro-6-[4-(1-imidazolyl)-phenyl]-3(2H)-pyridazinon ((CI 914, Drugs of the Future 9 (1984), 256) = B sind in der nachfolgenden Tabelle 1 wiedergegeben.

Tabelle 1

| Verbindung | Dosis | Kontraktilität | Herzschlagrate | Blutdruck |
|---|---|---|---|---|
| B | 0,01 | +8,7 | +3,6 | −1,3 |
| | 0,0316 | +25,5 | +18,9 | −0,7 |
| | 0,1 | +64,8 | +15,4 | −10,4 |
| A | 0,01 | +32,4 | +5,9 | −0,2 |
| | 0,0316 | +83,3 | +20,0 | −4,1 |
| | 0,1 | +185,5 | +32,1 | −17,4 |

Die Hemmung der Collagen-induzierten Thrombozytenaggregation in vitro wurde nach der Methode von Born (Nature, 194, 927–929 (1962) am thrombozytenreichen Humanplasma mit den erfindungsgemässen Substanzen 3,6-Dimethyl-5-[5-(1-imidazolyl)-2-thienyl]-2(1H)-pyridon-Hydrochlorid = C, 5-[4-(1-Imidazolyl)-2-thienyl]-6-methyl-2(1H)-pyridon-Hydrochlorid = D im Vergleich mit der Referenzsubstanz Acetylsalicylsäure = E durchgeführt.

Als $IC_{50}$ wird die Konzentration angegeben, welche eine 50%igen Hemmung der Aggregation verursachte.

Tabelle 2

| Substanz | $IC_{50}$ (Mol/l) |
|---|---|
| C | $4,4 \times 10^{-7}$ |
| D | $5,4 \times 10^{-7}$ |
| E | $9,0 \times 10^{-5}$ |

Die vorliegende Erfindung betrifft ebenfalls pharmazeutische Präparate, welche die neuen Verbindungen der Formel I in Form ihrer freien Verbindungen oder als Salze mit pharmazeutisch verträglichen Säuren oder Basen enthalten. Bei den erfindungsgemässen pharmazeutischen Präparaten handelt es sich um solche zur enteralen wie oralen oder rectalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragees, Kapseln, Suppositorien, Granulate, Lösungen, Emulsionen oder Suspensionen. Die Dosierung der Verbindungen liegt üblicherweise zwischen 0,1 und 500 mg pro Tag, vorzugsweise zwischen 0,5 und 100 mg pro Tag, und kann in einer Dosis oder mehreren Teildosen, vorzugsweise in zwei bis drei Teildosen pro Tag, verabreicht werden.

Die Herstellung der erfindungsgemässen Verbindungen wird durch die folgenden Beispiele näher erläutert. Die angegebenen Schmelzpunkte wurden mit einem Büchi-Schmelzpunktbestimmungsapparat gemessen und sind nicht korrigiert. Die IR-Spektren wurden mit dem Gerät Nicolet NIC-3600 und die Massenspektren mit dem Gerät Varian MAT-311A (70 eV) aufgenommen.

Beispiel 1

3,4-Dihydro-3,6-dimethyl-5-[4-(1-imidazolyl)-2-thienyl]-2(1H)-pyridon.

a) 1-[2-(1-Nitro-1-propenyl)-4-thienyl]-imidazol.

Eine Mischung aus 94 g 4-(1-Imidazolyl)-thiophen-2-aldehyd (EPA 112 987), 47,7 g Nitroethan, 4,9 g β-Alanin und 327 ml n-Butanol wird 9 Stunden bei Rückflusstemperatur gerührt. Anschliessend wird eingeengt und der Rückstand durch Säulenchromatographie (Kieselgel/Dichlormethan/Methanol) gereinigt.

Ausbeute: 47,7 g
Schmp. 158–159 °C
IR (in KBr): 1643, 1561, 1513, 1301 $cm^{-1}$
MS [m/e]: 235 ($M^+$, 100%), 188 (23%), 150 (27%).

b) 1-[4-(1-Imidazolyl)-2-thienyl]-2-propanon.

Eine Mischung aus 34 g 1-[2-(2-Nitro-1-propenyl)-4-thienyl]-imidazol, 65 g Eisenpulver, 0,9 g Eisentrichlorid-Heyahydrat, 51 ml Methanol und 100 ml Wasser wird unter Rühren auf Rückflusstemperatur gebracht. Bei dieser Temperatur werden 65 ml konzentrierte Salzsäure in 1 Stunde zugetropft. Anschliessend wird noch 1½ Stunden unter Rückfluss weitergerührt. Nach Abkühlen wird die Mischung in die 4-fache Menge Methanol eingerührt. Danach wird abfiltriert. Das Filtrat wird auf ca. ⅕ seines Volumens eingeengt und mit Wasser auf 500 ml aufgefüllt. Der dabei ausfallende braune Niederschlag wird abgesaugt, das Filtrat anschliessend mehrmals mit Dichlormethan extrahiert. Die vereinten Dichlormethanphasen werden getrocknet und eingeengt. Das zurückbleibende Rohprodukt wird ohne weitere Reinigung in die nächsten Reaktionsstufen eingesetzt.

Ausbeute: 10 g braunes Öl

c) 3,4-Dihydro-3,6-dimethyl-5-[4-(1-imidazolyl)-2-thienyl]-2(1H)-pyridon.

Analog Beispiel 1 wird die Reaktion durchgeführt mit 2 g Keton aus Beisp. 1b), 800 mg Methacrylsäureamid, 30 ml 1,4-Dioxan und 1,15 g Kalium-tert-butylat.

Ausbeute: 1,6 g
Schmp. 225 °C
IR (in KBr): 1678, 1636 $cm^{-1}$
MS [m/e: 273 ($M^+$, 100%), 258 (1%), 244 (2%), 230 (6%), 216 (9%), 202 (5%), 190 (30%), 175 (10%), 149 (3%).

Beispiel 2

3,4-Dihydro-5-[4-(1-imidazolyl)-2-thienyl]-6-methyl-2(1H)-pyridon.

Analog Beispiel 1 wird die Reaktion durchgeführt mit 2 g Keton aus Beisp. 1b), 810 mg Acrylsäureamid, 30 ml 1,4-Dioxan und 1,15 g Kalium-tert-butylat.

Ausbeute: 0,3 g
Schmp. 213–215 °C (Zers.)
IR (in KBr): 1680, 1639 $cm^{-1}$
MS [m/e]: 259 ($M^+$, 100%), 244 (4%), 230 (6%), 216 (10%), 190 (23%), 175 (8%).

Beispiel 3

3,4-Dihydro-4,6-dimethyl-5-[4-(1-imidazolyl)-2-thienyl]-2(1H)-pyridon.

a) 4-[4-(1-Imidazolyl)-2-thienyl]-3-methyl-5-oxohexansäurenitril.

Analog Beispiel 1a) wird die Reaktion durchgeführt mit 4,8 g Keton aus Beispiel 4b) und 5,8 ml Crotonsäurenitril. Das ölige Rohprodukt (2,3 g) wird ohne weitere Reinigung in die nächste Stufe eingesetzt.

b) 3,4-Dihydro-4,6-dimethyl-5-[4-(1-imidazolyl)-2-thienyl]-2(1H)-pyridon.

Analog Beispiel 1b) wird die Reaktion durchgeführt mit 1,8 g rohem Nitril aus Beispiel 6a), 18 ml Essigsäure und 2,7 ml konzentrierter Schwefelsäure.

Ausbeute: 0,7 g
Schmp. 178–179 °C
IR (in KBr): 1681 $cm^{-1}$
MS [m/e]: 273 ($M^+$, 100%), 258 (76%), 230 (6%).

Beispiel 4

3,4-Dihydro-3,6-dimethyl-5-[5-(1-imidazolyl)-2-thienyl]-2(1H)-pyridon.

a) 1-[5-(2-Nitro-1-propenyl)-2-thienyl]-imidazol.

Analog Beispiel 1a) wird die Umsetzung durchgeführt mit 60 g 5-(1-Imidazolyl)-thiophen-2-aldehyd (EPA 112 987), 23,1 g Nitroethan, 2,4 g β-Alanin und 210 ml n-Butanol. Ausbeute: 40,7 g
Schmp. 145–147 °C
IR (in KBr): 1631, 1543, 1512, 1304 $cm^{-1}$
MS [m/e]: 235 ($M^+$, 27%), 178 (100%), 168 (16%), 150 (29%), 123 (21%).

b) 1-[5-(1-Imidazolyl)-2-thienyl]-2-propanon.

Analog Beispiel 4b) wird die Reaktion durchgeführt mit 35 g aus Beispiel 4a), 73,6 g Eisenpulver, 1 g Eisentrichlorid-Hexahydrat, 60 ml Methanol, 125 ml Wasser und 75 ml konzentrierter Salzsäure.

Ausbeute: 10,7 g Öl
IR (in substanz): 1713 $cm^{-1}$
MS [m/e]: 206 ($M^+$, 27%), 164 (21%), 163 (100%), 136 (16%), 109 (20%).

c) 3,4-Dihydro-3,6-dimethyl-5-[5-(1-imidazolyl)-2-thienyl]-2(1H)-pyridon.

Analog Beispiel 1 wird die Reaktion durchgeführt mit 10,7 g Keton aus Beispiel 4b), 4,8 g Methacrylsäureamid, 5,8 g Kalium-tert-butylat und 100 ml 1,4-Dioxan.

Ausbeute: 6,3 g
Schmp. 136 °C
IR (in KBr): 1671, 1634 $cm^{-1}$
MS [m/e]: 273 ($M^+$, 100%), 258 (1%), 230 (8%), 150 (23%), 149 (23%).

Beispiel 5

1,2-Dihydro-5-[4-(1-imidazolyl)-2-thienyl]-6-methyl-2-oxo-pyridin-3-carbonsäurenitril.

Eine Mischung aus 7 g Keton aus Beisp. 1b), 70 ml N,N-Dimethylformamid-dimethylacetal und 120 ml Acetonitril wird 18 Stunden bei Raumtemperatur gerührt. Anschliessend wird eingeengt. Der Rückstand wird mit 140 ml Dimethylformamid, 7 g Cyanacetamid und 8,4 g Natriummethylat versetzt und die erhaltene Mischung 1½ Stunden zum Rückfluss erhitzt. Danach wird eingeengt, der Rückstand mit 120 ml Methanol und 40 ml Eisessig versetzt und 20 Minuten bei Raumtemperatur gerührt. Der ausgefallene Feststoff wird abgesaugt und anschliessend säulenchromatographisch (Kieselgel/Dichlormethan/Methanol) gereinigt.

Ausbeute: 3,5 g
Schmp. 300 °C (Zers.)
IR (in KBr): 2225, 1679 $cm^{-1}$
MS [m/e]: 282 ($M^+$, 100%), 254 (4%), 228 (5%).

Beispiel 6

1,2-Dihydro-5-[5-(1-imidazolyl)-2-thienyl]-6-methyl-2-oxo-pyridin-3-carbonsäurenitril.

Analog Beispiel 5 wird die Umsetzung durchgeführt mit 2,3 g Keton aus Beispiel 4b), 2,3 ml N,N-Dimethylformamid-dimethylacetal, 20 ml Acetonitril, 40 ml Dimethylformamid, 2,1 g Cyanacetamid und 2,7 g Natriummethylat.

Ausbeute: 0,96 g
Schmp. 277–280 °C
IR (in KBr): 2223, 1672 $cm^{-1}$
MS [m/e]: 282 ($M^+$, 100%), 267 (3%), 249 (9%), 228 (14%).

Beispiel 7

1,2-Dihydro-5-[4-(1-imidazolyl)-2-thienyl]-6-methyl-2-oxo-pyridin-3-carbonsäure.

3,5 g Nitril aus Beispiel 5 werden 5 Stunden in 50 ml konzentrierter Salzsäure bei Rückflusstempertur gerührt. Nach Abkühlen wird mit Natronlauge neutralisiert, der ausgefallene Feststoff abgesaugt, mit Wasser gewaschen und aus Dimethylformamid/Wasser unter Verwendung von Aktivkohle umkristallisiert.

Ausbeute: 2,4 g
Schmp. 298–300 °C (Zers.)
IR (in KBr): 1737, 1641 $cm^{-1}$
MS [m/e]: 301 ($M^+$, 30%), 257 (100%), 229 (12%).

Beispiel 8

1,2-Dihydro-5-[5-(1-imidazolyl)-2-thienyl]-6-methyl-2-oxo-pyridin-3-carbonsäure.

Analog Beispiel 7 wird die Reaktion durchgeführt mit 0,66 g Nitril aus Beispiel 6 und 20 ml konzentrierter Salzsäure.

Ausbeute: 0,58 g
Schmp. 278 °C (Zers.)
IR (in KBr): 1732, 1639 $cm^{-1}$
MS [m/e]: 301 ($M^+$, 100%), 283 (37%), 257 (35%), 242 (4%), 216 (13%).

Beispiel 9

5-[4-(1-Imidazolyl)-2-thienyl]-6-methyl-2(1H)-pyridon.

1 g Säure aus Beispiel 7 wird in 30 ml Diphenylether 2 Stunden bei Rückflusstemperatur gerührt. Nach Abkühlen wird die Mischung zwischen Dichlormethan und 7%iger Salzsäure verteilt. Die wässrige Phase wird mehrmals mit Dichlormethan gewaschen und danach durch Zugabe von Natronlauge auf pH 7 eingestellt. Der dabei ausgefallene Feststoff wird abgesaugt, mit Wasser gewaschen und getrocknet. Zur Reinigung wird er in einer Mischung aus Dichlormethan und Methanol gelöst. Nach Zugabe von Aktivkohle wird kurz aufgekocht und die Mischung durch eine Kieselgel-Schicht filtriert. Der beim Einengen des Filtrats ausgefallene Feststoff wird abgesaugt und getrocknet.

Ausbeute: 280 mg
Schmp. 230 °C
IR (in KBr): 1659 $cm^{-1}$
MS [m/e]: 257 ($M^+$, 100%), 242 (3%), 229 (12%), 203 (4%), 187 (5%).

Beispiel 10

5-[-5-(1-Imidazolyl)-2-thienyl]-6-methyl-2(1H)-pyridon.

Analog Beispiel 9 wird die Umsetzung durchgeführt mit 0,57 g Säure aus Beispiel 8 und 20 ml Diphenylether.

Ausbeute: 0,18 g
Schmp. 233°C
IR (in KBr): 1656 $cm^{-1}$
MS [m/e]: 257 ($M^+$, 100%), 242 (2%), 229 (4%), 203 (4%), 187 (5%).

Beispiel 11

3,6-Dimethyl-5-[4-(1-imidazolyl)-2-thienyl]-2(1H)-pyridon.

Eine Mischung aus 1,08 g 3,4-Dihydropyridon aus Beispiel 1c), 1,14 g 3-Nitrobenzolsulfonsäure-Natriumsalz, 0,8 g Natriumhydroxid und 25 ml Wasser wird 20 Stunden bei Rückflusstemperatur gerührt. Nach Abkühlen wird mit Essigsäure neutralisiert, der ausgefallene Feststoff abgesaugt, getrocknet und durch Säulenchromatographie (Kieselgel/Dichlormethan/Methanol) gereinigt.

Ausbeute: 0,25 g
Schmp. 250°C (Zers.)
IR (in KBr): 1658 $cm^{-1}$
MS [m/e]: 271 ($M^+$, 100%), 256 (5%), 243 (11%), 242 (12%), 201 (6%), 174 (4%), 160 (4%), 135 (5%), 108 (6%).

Beispiel 12

3,6-Dimethyl-5-[4-(1-imidazolyl)-2-thienyl]-2(1H)-pyridon.

Eine Mischung von 2,5 g 3,4-Dihydropyridon aus Beispiel 1c), 0,5 g Schwefelpulver und 50 ml einer eutektischen Mischung aus Diphenyl und Diphenylether (Dowtherm A) wird 2 Stunden auf 190–200°C erhitzt. Nach Abkühlen auf Raumtemperatur wird mit Ether/verdünnter Salzsäure geschüttelt, die salzsaure Phase mit Ammoniumhydroxid-Lösung neutralisiert, der ausgefallene Feststoff abgesaugt, getrocknet und durch Säulenchromatographie (Kieselgel/Dichlormethan/Methanol) gereinigt.

Ausbeute: 1g
Schmp. 252°C (Zers.)

Beispiel 13

3,6-Dimethyl-5-[5-(1-imidazolyl)-2-thienyl]-2(1H)-pyridon.

Analog Beispiel 12 wird die Reaktion durchgeführt mit 4 g 3,4-Dihydropyridon aus Beispiel 4c), 0,8 g Schwefelpulver und 80 ml der eutektischen Mischung aus Diphenyl und Diphenylether.

Ausbeute: 0,7 g
Schmp. 202–204°C (Zers.)
IR (in KBr): 1645 $cm^{-4}$
MS [m/e]: 271 ($M^+$, 100%), 256 (1%), 243 (2%), 242 (3%), 201 (3%), 174 (2%), 160 (2%), 135 (2%).

Beispiel 14

4,6-Dimethyl-5-[4-(1-imidazolyl)-2-thienyl]-2(1H)-pyridon.

2 g Keton aus Beispiel 1b) werden in 5 ml Methansulfonsäure gelöst. Dazu werden 0,68 g Acetessigsäureamid und 5,4 g Polyphosphorsäure gegeben und die Mischung 90 min auf 120–130°C erhitzt. Nach Abkühlen wird die Reaktionsmischung in Eiswasser eingerührt und mit Ammoniumhydroxid-Lösung neutralisiert. Anschliessend wird mit Dichlormethan extrahiert, die Dichlormethanphase getrocknet, eingeengt und der Rückstand durch Säulenchromatographie (Kieselgel)/Dichlormethan/Methanol) gereinigt.

Ausbeute: 0,3 g
Schmp. 243°C (Zers.)
IR (in KBr): 1656 $cm^{-1}$
MS [m/e]: 271 ($M^+$, 100%), 256 (1%), 243 (16%), 228 (3%), 201 (4%), 136 (4%).

Analog Beispiel 14 werden hergestellt:

3,4-Dihydro-3,6-dimethyl-5-[4-(1-imidazolyl)-2-thienyl]-2(1H)-pyridon-hydrochlorid mit Schmp. 245–248°C (Zers.)

1,2-Dihydro-5-[4-(1-imidazolyl)-2-thienyl]-6-methyl-2-oxo-pyridin-3-carbonsäurenitril-hydrochlorid mit Schmp. 318°C (Zers.)

1,2-Dihydro-5-[4-(1-imidazolyl)-2-thienyl]-6-methyl-2-oxo-pyridin-3-carbonsäure-hydrochlorid mit Schmp. 294-296°C (Zers.)

5-[4-(1-Imidazolyl)-2-thienyl]-6-methyl-2(1H-pyridon-hydrochlorid mit Schmp. 287–289°C (Zers.)

3,6-Dimethyl-5-[4-(1-imidazolyl)-2-thienyl]-2(1H)-pyridon-hydrochlorid mit Schmp. 306°C (Zers.)

3,4-Dihydro-4,6-dimethyl-5-[4-(1-imidazolyl)-2-thienyl]-2(1H)-pyridon-hydrochlorid mit Schmp. 140°C (Zers.)

3,4-Dihydro-3,6-dimethyl-5-[5-(1-imidazolyl)-2-thienyl]-2(1H)-pyridon-hydrochlorid mit Schmp. 273°C (Zers.)

1,2-Dihydro-5-[5-(1-imidazolyl)-2-thienyl]-6-methyl-2-oxo-pyridon-3-carbonsäurenitril-hydrochlorid mit Schmp. 310°C (Zers.)

5-[5-(1-Imidazolyl)-2-thienyl]-6-methyl-2(1H)-pyridon-hydrochlorid mit Schmp. 287°C (Zers.)

3,6-Dimethyl-5-[5-(1-imidazolyl)-2-thienyl]-2(1H)-pyridon-hydrochlorid mit Schmp. 298°C (Zers.)

4,6-Dimethyl-5-[4-(1-imidazolyl)-2-thienyl-2(1H)-pyridon-hydrochlorid mit Schmp. 310°C (Zers.)

**Patentansprüche für die Vertragsstaaten: BE, CH, DE, FR, GB, IT, LI, LU, NL, SE**

1. 3,4-Dihydro-2(1H)-pyridone der Formel I,

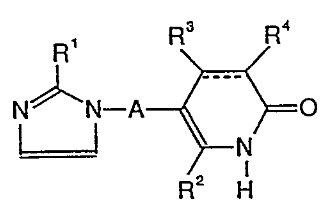

I

in der
═══ eine Einfachbindung zwischen zwei Kohlenstoffatomen bedeutet,
$R^1$, $R^3$, $R^4$ unabhängig voneinander Wasserstoff oder einen Methylrest,
$R^2$ einen $C_{1-4}$-Niederalkylrest bedeuten und für Thienylen steht.

2. 3,4-Dihydro-2(1H)-pyridone der Formel I, in der
═══ eine Einfachbindung zwischen zwei Kohlenstoffatomen bedeutet,
$R^1$, $R^3$, $R^4$ unabhängig voneinander Wasserstoff oder einen Methylrest,
$R^2$ einen $C_{1-4}$-Niederalkylrest bedeuten und

A für 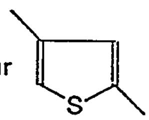 oder 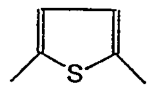 steht.

3. 2(1H)-pyridone der Formel I, in der
═══ eine Doppelbindung zwischen zwei Kohlenstoffatomen bedeutet,

A für 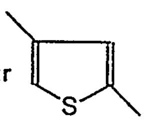 oder 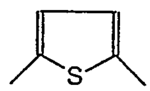 steht,

$R^1$, $R^3$, unabhängig voneinander Wasserstoff oder einen Methylrest,
$R^2$ einen $C_{1-4}$-Niederalkylrest und
$R^4$ Wasserstoff, Methyl, -COOH, -CN, -$CONH_2$, -$NH_2$ bedeuten.

4. Verfahren zur Herstellung von 3,4-Dihydro-2(1H)-pyridonen der Formel I gemäss den Ansprüchen 1 und 2, dadurch gekennzeichnet, dass man

a) ein 5-Oxo-pentansäurenitril der Formel II

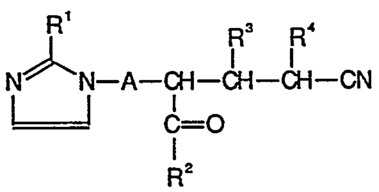 II

in der A, $R^1$, $R^2$, $R^3$, $R^4$ die in Formel I, Ansprüche 1 und 2 angegebenen Bedeutungen haben, mit starken Säuren behandelt, oder

b) einen 5-Oxo-pentansäureester der Formel III

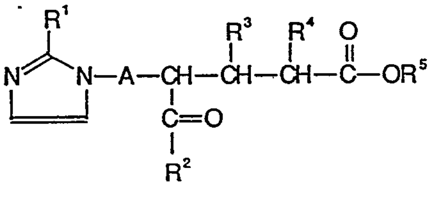 III

in der A, $R^1$, $R^2$, $R^3$, $R^4$ die in Formel I, Ansprüche 1 und 2 angegebenen Bedeutungen haben und $R^5$ für einen $C_{1-4}$-Alkylrest steht, mit Ammoniak oder einem geeigneten Ammoniumsalz behandelt, oder

c) ein Alkanon der Formel IV

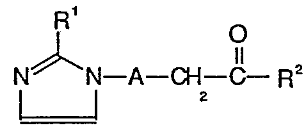 IV

in der A, $R^1$, $R^2$ die in Formel I, Ansprüche 1 und 2 angegebenen Bedeutungen haben, in einem inerten Lösungsmittel mit einem Acrylsäureamid der Formel V,

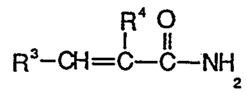 V

in der $R^3$, $R^4$ die in Formel I, Ansprüche 1 und 2 angegebenen Bedeutungen haben, in Gegenwart von Kalium-tert-butylat oder einer anderen geeigneten Base umsetzt.

5. Verfahren zur Herstellung von 2(1H)-Pyridonen der Formel I gemäss dem Anspruch 3 mit $R^4$ = H, $CH_3$, dadurch gekennzeichnet, dass man 3,4-Dihydro-2(1H)-pyridone der Formel I gemäss den Ansprüchen 1 und 2 mit Palladium, Schwefel, 3-Nitrobenzolsulfonsäure, Brom/-Eisessig oder einem anderen geeigneten Dehydrierungsmittel umsetzt.

6. Verfahren zur Herstellung von 2(1H)-pyridonen der Formel I gemäss dem Anspruch 3 mit $R^4$ = H, COOH, CN, $CONH_2$, $HN_2$, dadurch gekennzeichnet, dass man Alkanone der Formel IV, in der A, $R^1_1$, $R^2$ die in Formel I, Anspruch 3 angegebenen Bedeutungen haben, mit einem gegebenfalls alkylsubstituierten N,N-Dimethyl-formamid-dimethylacetal der Formel VI

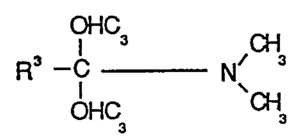 VI

in der $R^3$ die in Formel I angegebene Bedeutung hat, zu den 2-(Dimethylamino)-ethenyl-aklanonen der Formel VII umsetzt.

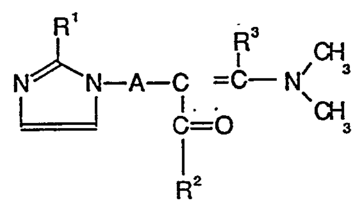 VII

in der A, $R^1$, $R^2$, $R^3$ die in Formel I angegebenen Bedeutungen haben, diese Verbindungen der Formel VII unter basischen Bedingungen mit Cyanacetamid zu den 2(1H)-Pyridonen der Formel I mit $R^4$ = CN umsetzt und, falls erwünscht, diese Nitrile der Formel I durch Hydrolyse in die entsprechende Säureamide der Formel I

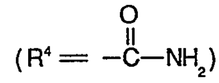

oder Säuren der Formel I ($R^4$ = COOH), diese durch thermische Decarboxylierung in die ent-

sprechenden Verbindungen der Formel I mit $R^4$ = H, oder die Säureamide der Formel I durch Hofmann-Abbau in die entsprechenden Amine der Formel I ($R^4 = NH_2$) überführt.

7. Pharmazeutische Präparate, dadurch gekennzeichnet, dass sie eine Verbindung der Formel I gemäss den Ansprüchen 1–3 oder deren Salze mit physiologisch unbedenklichen Säuren bzw. Basen als Wirkstoff im Gemisch mit üblichen pharmazeutischen Hilfs- oder Trägerstoffen enthalten.

**Patentansprüche für den Vertragsstaat: AT**

1. Verfahren zur Herstellung von 3,4-Dihydro-2(1H)-pyridonen der Formel I,

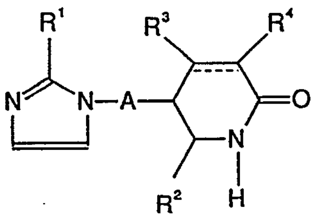

I

in der
------ eine Einfachbindung zwischen zwei Kohlenstoffatomen bedeutet,
$R^1$, $R^2$, $R^4$ unabhängig voneinander Wasserstoff oder einen Methylrest,
$R^2$ einen $C_{1-4}$-Niederalkylrest bedeuten und
A für Thienylen steht, dadurch gekennzeichnet, dass man

a) ein 5-Oxo-pentansäurenitril der Formel II

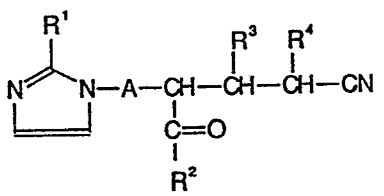

II

in der A, $R^1$, $R^2$, $R^3$, $R^4$ die in Formel I angegebenen Bedeutungen haben, mit starken Säuren behandelt, oder

b) einen 5-Oxo-pentansäureester der Formel III

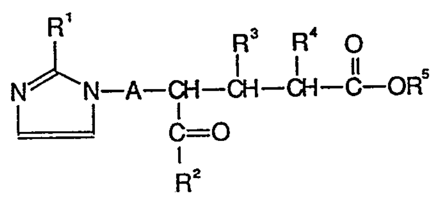

III

in der A, $R^1$, $R^2$, $R^3$, $R^4$ die in Formel I, angegebenen Bedeutungen haben und $R^5$ für einen $C_{1-4}$-Alkylrest steht, mit Ammoniak oder einem geeigneten Ammoniumsalz behandelt, oder

c) ein Alkanon der Formel IV

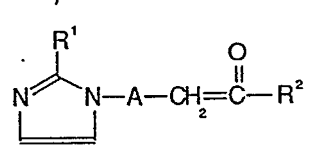

IV

in der A, $R^1$, $R^2$ die in Formel I angegebene Bedeutung haben, in einem inerten Lösungsmittel mit einem Acrylsäureamid der Formel V,

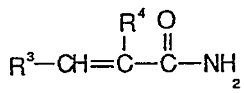

V

in der $R^3$, $R^4$ die in Formel I angegebenen Bedeutungen haben, in Gegenwart von Kalium-tert-butylat oder einer anderen geeigneten Base umsetzt, und, falls erwünscht, man erhaltene 3,4-Dihydro-2(1H)-pyridone der Formel I mit Palladium, Schwefel, 3-Nitrobenzolsulfonsäure, Brom/-Eisessig oder einem anderen geeigneten Dehydrierungsmittel umsetzt.

2. Verfahren zur Herstellung von 2(1H)-pyridonen der Formel I mit $R^4$ = H, COOH, CN, $CONH_2$, $NH_2$, dadurch gekennzeichnet, dass man Alkanone der Formel IV, in der A, $R^1$, $R^2$ die in Anspruch 1 angegebenen Bedeutungen haben, mit einem gegebenenfalls alkylsubstituierten N,N-Dimethyl-formamid-dimethylacetal der Formel VI

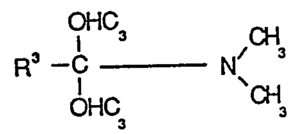

VI

in der $R^3$ die in Formel I angegebene Bedeutung hat, zu den 2-(Dimethylamino)-ethenyl-aklanonen der Formel VII umsetzt.

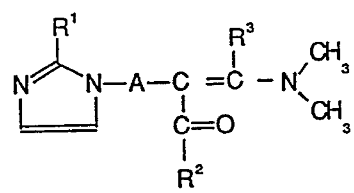

VII

in der A, $R^1$, $R^2$, $R^3$ die in Formel I angegebenen Bedeutungen haben, diese Verbindungen der Formel VII unter basischen Bedingungen mit Cyanacetamid zu den 2(1H)-Pyridonen der Formel I mit $R^4$ = CN umsetzt und, falls erwünscht, diese Nitrile der Formel I durch Hydrolyse in die entsprechende Säureamide der Formel I

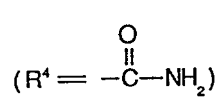

oder Säuren der Formel I ($R^4$ = COOH), diese durch thermische Decarboxylierung in die entsprechenden Verbindungen der Formel I mit $R^4$ = H, oder die Säureamide der Formel I durch Hofmann-Abbau in die entsprechenden Amine der Formel I ($R^4 = NH_2$) überführt.

**Revendications pour les Etats contractants BE, CH, DE, FR, GB, IT, LI, LU, NL, SE**

1. 3,4-dihydro-2(1H)-pyridones de formule I

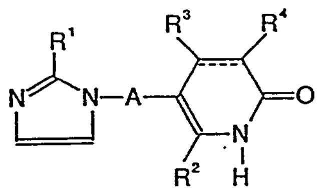

I

dans laquelle
------ représente une liaison simple entre deux atomes de carbone,
$R^1$, $R^3$, $R^4$ représentent chacun, indépendamment les uns des autres, l'hydrogène ou un groupe méthyle,
$R^2$ représente un groupe alkyle inférieur en C1-C4, et
A représente un groupe thiénylène.

2. 3,4-dihydro-2(1H)-pyridones de formule I dans laquelle
------ représente une liaison simple entre deux atomes de carbone,
$R^1$, $R^3$, $R^4$ représentent chacun, indépendamment les uns des autres, l'hydrogène ou un groupe méthyle,
$R^2$ représente un groupe alkyle inférieur en C1-C4, et

A représente 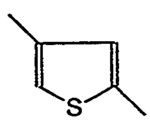 ou 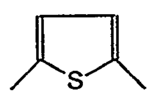

3. 2(1H)-pyridones de formule I dans laquelle
------ représente une double liaison entre deux atomes de carbone,

A représente 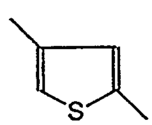 ou 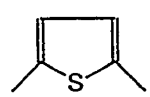

$R^1$, $R^3$ représentent chacun, indépendamment l'un de l'autre, l'hydrogène ou une groupe méthyle,
$R^2$ représente un groupe alkyle inférieur en C1-C4, et
$R^4$ représente l'hydrogène, un groupe méthyle, -COOH, -CN, $-CONH_2$, $-NH_2$.

4. Procédé de préparation des 3,4-dihydro-2(1H)-pyridones de formule I selon les revendications 1 et 2, caractérisé en ce que:

a) on traite un 5-oxo-pentanonitrile de formule II

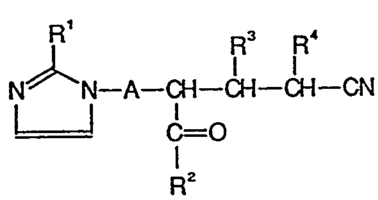

II

dans laquelle A, $R^1$, $R^2$, $R^3$, $R^4$ ont les significations indiquées dans la formule I, revendications 1 et 2, par des acides forts, ou bien

b) on traite un ester 5-oxo-pentanoïque de formule III

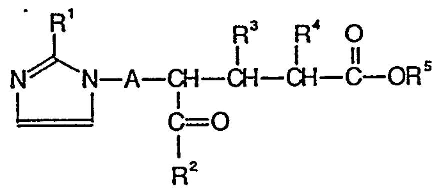

III

dans laquelle A, $R^1$, $R^2$, $R^3$, $R^4$ ont les significations indiquées en référence à la forumle I, revendications 1 et 2, et $R^5$ représente un groupe alkyle en C1-C4, par l'ammoniac ou sel d'ammonium approprié, ou bien

c) on fait réagir une alcanone de formule IV

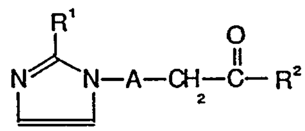

IV

dans laquelle A, $R^1$, $R^2$ ont les significations indiquées en référence à la formule I, revendications 1 et 2, dans un solvant inerte, par un acrylamide de formule V

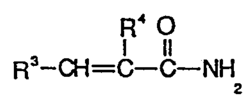

V

dans laquelle $R^3$, $R^4$ ont les significations indiquées en référence à la formule I, revendications 1 et 2, en présence de tert-butylate de potassium ou d'une autre base appropriée.

5. Procédé de préparation des 2(1H)-pyridones de forumle I selon la revendication 3, dans lesquelles $R^4$ = H, CH, caractérisé en ce que l'on fait réagir des 3,4-dihydro-2(1H)-pyridones de formule I selon les revendications 1 et 2 avec du palladium, du soufre, l'acide 3-nitrobenzène-sulfonique, le couple brome/acide acétique glacial ou un autre agent déshydrogénant approprié.

6. Procédé de préparation des 2(1H)-pyridones de formule I selon la revendication 3, dans lesquelles $R^4$ = H, COOH, CN, $CONH_2$, $NH_2$, caractérisé en ce que l'on fait réagir des alcanones de formule IV dans laquelle A, $R^1$, $R^2$ ont les significations indiquées en référence à la formule I dans la revendication 3, avec un diméthylacétal du N,N-diméthylformamide portant éventuellement des substituants alkyle et répondant à la formule VI

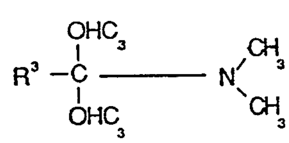

VI

dans laquelle $R^3$ a les significations indiquées en référence à la formule I, ce qui donne les 2-(diméthylamino)-éthényl-alcanones de formule VII

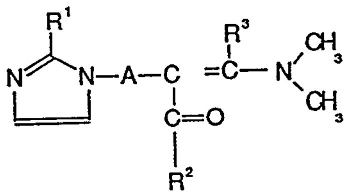

VII

dans laquelle A, $R^1$, $R^2$, $R^3$ ont les significations indiquées en référence à la formule I, qu'on fait réagir en milieu basique avec le cyanacétamide, ce qui donne les 2(1H)-pyridones de formule I dans laquelle $R^4$ = CN qu'on convertit si on le désire par hydrolyse en les amides correspondants de formule I

$$(R^4 = -\overset{O}{\overset{\|}{C}}-NH_2)$$

ou en les acides de formule I ($R^4$ = COOH), et ces derniers, par décarboxylation à la chaleur, en les composés correspondants de formule I dans laquelle $R^4$ = H, ou bien les amides de formule I, par und dégradation d'Hofmann, en les amines correspondantes de formule I ($R^4 = NH_2$).

7. Compositions pharmaceutiques, caractérisées en ce qu'elles contiennent un composé de formule I selon les revendications 1 à 3 ou un sel d'un tel composé et d'un acide ou d'une base acceptable pour l'usage pharamceutique, en tant que substance active, en mélange avec des produits auxiliaires ou véhicules pharmaceutiques usuels.

**Revendications pour l'Etat contractant AT**

1. Procédé de préparation des 3,4-dihydro-2(1H)-pyridones de formule I

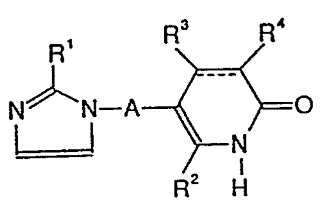

I

dans laquelle

--- représente une liaison simple entre deux atomes de carbone,

$R^1$, $R^3$, $R^4$ représentent chacun, indépéndamment les uns des autres, l'hydrogène ou un groupe méthyle,

$R^2$ représente un groupe alkyle inférieur en C1-C4, et

A représente un groupe thiénylène, caractérisé en ce que:

a) on traite un 5-oxo-pentanonitrile de formule II

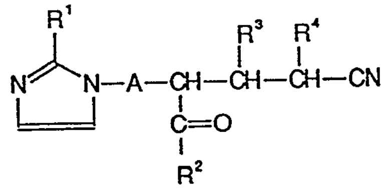

II

dans laquelle A, $R^1$, $R^2$, $R^3$, $R^4$ ont les significations indiquées en référence à la formule I, par des acides forts, ou bien

b) on traite un ester 5-oxo-pentanoïque de formule III

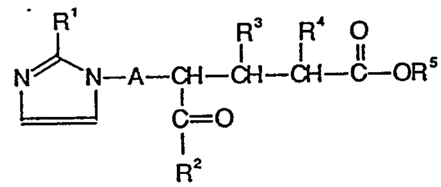

III

dans laquelle A, $R^1$, $R^2$, $R^3$, $R^4$ ont les significations indiqueés en référence à la formule I et $R^5$ représente un groupe alkyle en C1-C4, par l'ammoniac ou un sel d'ammonium approprié, ou bien

c) on fait réagir une alcanone de formule IV

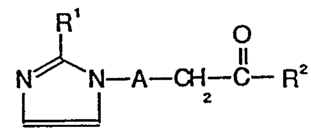

IV

dans laquelle A, $R^1$, $R^2$ ont les significations indiquées en référence à la formule I, dans un solvant inerte, avec un acrylamide de formule V

$$R^3-CH=\overset{R^4}{\overset{|}{C}}-\overset{O}{\overset{\|}{C}}-NH_2 \qquad V$$

dans laquelle $R^3$, $R^4$ ont les significations indiquées en référence à la formule I, en présence de tert-butylate de potassium ou d'une autre base appropriée, ce qui donne une 3,4-dihydro-2(1H)-pyridone de formule I qu'on fait réagir si on le désire avec le palladium, le soufre, l'acide 3-nitrobenzène-sulfonique, le couple brome/acide acétique glacial ou un autre agent déshydrogénant approprié.

2. Procédé de préparation des 2(1H)-pyridones de formule I dans lesquelles $R^4$ = H, COOH, CN, $CONH_2$, $NH_2$, caractérisé en ce que l'on fait réagir des alcanones de formule IV dans laquelle A, $R^1$, $R^2$ ont les significations indiquées dans la revendication 1, avec un diméthylacétal du N,N-diméthylformamide portant éventuellement des substituants alkyle et répondant à la formule VI

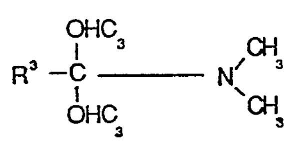

VI

dans laquelle $R^3$ a les significations indiquées en référence à la formule I, ce qui donne les 2-(diméthylamino)-éthényl-alcanones de formule VII

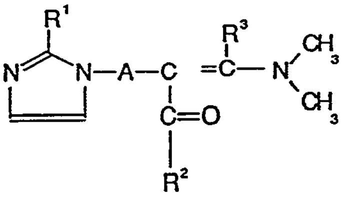

VII

dans laquelle A, $R^1$, $R^2$, $R^3$ ont les significations indiquées en référence à la formule I, qu'on fait réagir elles-mêmes en milieu basique avec le cyanacétamide, ce qui donne les 2(1H)-pyridones de formule I dans laquelle $R^4$ = CN, après quoi, si on le désire, on convertit ces nitriles de formule I par hydrolyse en les amides correspondants de formule I

$$(R^4 = -\overset{O}{\overset{\|}{C}}-NH_2)$$

ou les acides de formule I ($R^4$ = COOH), qu'on convertit eux-mêmes par décarboxylation à la chaleur en les composés correspondants de formule I dans laquelle $R^4$ = H, ou bien on convertit les amides de formule I par une dégradation d'Hofmann en les amines correspondantes de formule I ($R^4 = NH_2$).

**Claims for the contracting statey BE, CH, DE, FR, GB, IT, LI, LU, NL, SE**

1. 3,4-Dihydro-2(1H)-pyridones of the formula I

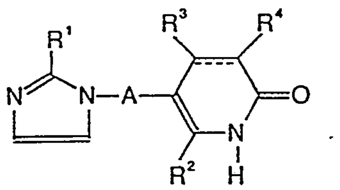

I

wherein
------ is a single bond between two carbon atoms,
$R^1$, $R^3$, $R^4$ independent from each other are hydrogen or a methyl radical,
$R^2$ is a $C_{1-4}$-lower alkyl radical and
A is thienylene.

2. 3,4-Dihydro-2(1H)-pyridones of the formula I, wherein
------ is a single bond between two carbon atoms,
$R^1$, $R^3$, $R^4$ independent from each other are hydrogen or a methyl radical,
$R^2$ is a $C_{1-4}$-lower alkyl radical and

A represents 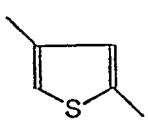 or 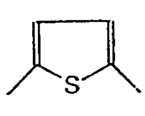

3. 2(1H)-Pyridones of the formula I, wherein
------ is a double bond between two carbon atoms,

A represents 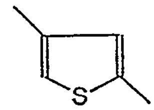 or 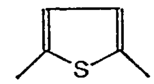

$R^1$, $R^3$ independent from each other are hydrogen or a methyl radical,
$R^2$ is a $C_{1-4}$-lower alkyl radical and
$R^4$ is hydrogen, methyl, -COOH, -CN, $-CONH_2$, $-NH_2$.

4. A process for the preparation of 3,4-dihydro-2(1H)-pyridones of the formula I according to claims 1 and 2, characterized in that

a) a 5-oxo-pentanoic acid nitrile of the formula II

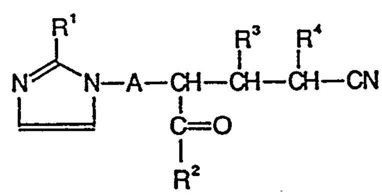

II

wherein A, $R^1$, $R^2$, $R^3$, $R^4$ have the meanings as defined in formula I, claims 1 and 2, is treated with strong acids, or

b) a 5-oxo-pentanoic acid ester of the formula III

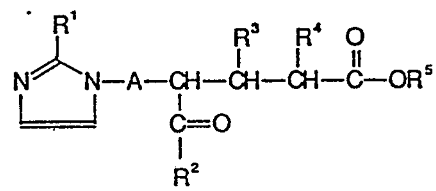

III

wherein A, $R^1$, $R^2$, $R^3$, $R^4$, have the meanings as defined in formula I, claims 1 and 2, and wherein $R^5$ represents a $C_{1-4}$-alkyl radical, is treated with ammonia or a suitable ammonium salt, or

c) an alkanone of the formula IV

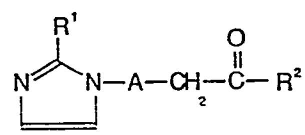

IV

wherein A, $R^1$, $R^2$ have the meanings as defined in formula I, claims 1 and 2, is reacted in an inert solvent with an acrylic acid amide of the formula V

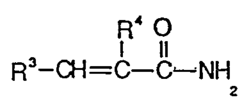

V

wherein $R^3$, $R^4$ have the meanings as defined in formula I, claims 1 and 2, in the presence of potassium tert.-butylate or of another suitable base.

5. A process for the preparation of 2(1H)-pyridones of the formula I according to claim 3, wherein $R^4$ means H, $CH_3$, characterized in that 3,4-dihydro-2(1H)-pyridones of the formula I according

to claims 1 and 2 are reacted with palladium, sulfur, 3-nitrobenzene sulfonic acid, bromine/glacial acetic acid or another suitable dehydrogenating agent.

6. A process for the preparation of 2(1H)-pyridones of the formula I according to claim 3, wherein $R^4$ means H, COOH, CN, $CONH_2$, $NH_2$, characterized in that alkanones of the formula IV, wherein A, $R^1$, $R^2$ have the meanings as defined in formula I, claim 3, are reacted with an optionally alkyl-substituted N,N-dimethyl-formamidedimethylacetal of the formula VI

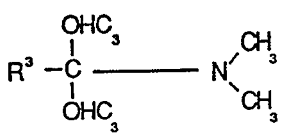

VI

wherein $R^3$ has the meaning as defined in formula I, to form the 2-(dimethylamino)-ethenyl-alkanones of the formula VII

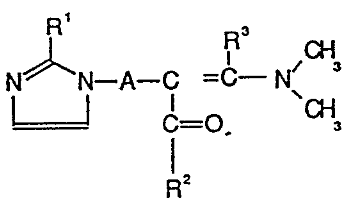

VII

wherein A, $R^1$, $R^2$, $R^3$ have the meanings as defined in formula I, that these compounds of the formula VII are reacted with cyanoacetamide under basic conditions to form the 2(1H)-pyridones of the formula I, wherein $R^4$ means CN, and, if desired, that these nitriles of the formula I are converted into the corresponding acid amides of the formula I

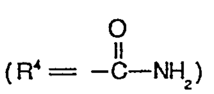

or acids of the formula I ($R^4$ = COOH) by hydrolysis, that these compounds are converted by thermic decarboxylation into the corresponding compounds of the formula I, wherein $R^4$ = H, or that the acid amides of the formula I are converted into the corresponding amines of the formula I ($R^4$ = $NH_2$) by the Hofmann-decomposition.

7. Pharmaceutical preparations, characterized in that they contain a compound of the formula I according to claims 1 to 3 or salts thereof with physiologically acceptable acids and bases, respectively, as active ingredient in mixture with common pharmaceutical excipients or carriers.

**Claims for contracting state AT**

1. A process for the preparation of 3,4-dihydro-2(1H)-pyridones of the formula I

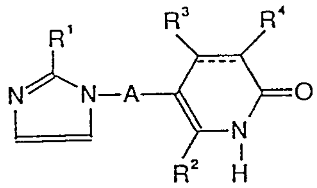

I

wherein

is a single bond between two carbon atoms,

$R^1$, $R^3$, $R^4$ independent from each other are hydrogen or a methyl radical,

$R^2$ is a $C_{1-4}$-lower alkyl radical and

A is thienylene,

characterized in that

a) a 5-oxo-pentanoic acid nitrile of the formula II

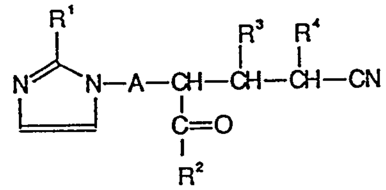

II

wherein A, $R^1$, $R^2$, $R^3$, $R^4$ have the meanings as defined in formula I, claims 1 and 2, is treated with strong acids, or

b) a 5-oxo-pentanoic acid ester of the formula III

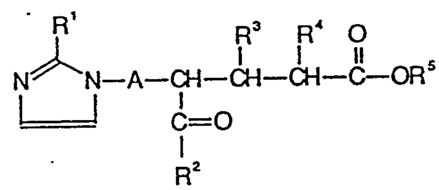

III

wherein A, $R^1$, $R^2$, $R^3$, $R^4$, have the meanings as defined in formula I, and wherein $R^5$ represents a $C_{1-4}$-alkyl radical, is treated with ammonia or a suitable ammonium salt, or

c) an alkanone of the formula IV

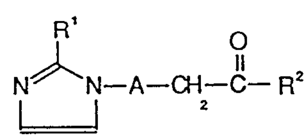

IV

wherein A, $R^1$, $R^2$ have the meanings as defined in formula I, is reacted in an inert solvent with an acrylic acid amide of the formula V

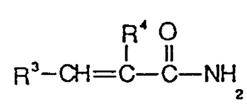

V

wherein $R^3$, $R^4$ have the meanings as defined in formula I, in the presence of potassium-tert-butylate or of another suitable base and, if desired, the resulting 3,4-dihydro-2(1H)-pyridones of formula I are subjected to reaction with palladium, sulfur, 3-nitrobenzene sulfonic acid, bromine/glacial acetic acid or another suitable dehydrogenating agent.

2. A process for the preparation of 2(1H)-pyridones of the formula I, wherein $R^4$ means H, COOH, CN, $CONH_2$, $NH_2$, characterized in that alkanones of the formula IV, wherein A, $R^1$, $R^2$ have the meanings as defined in claim 1, are reacted with an optionally alkyl-substituted N,N-dimethyl-formamide-dimethylacetal of the formula VI

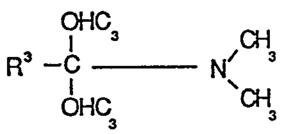

VI

wherein $R^3$ has the meaning as defined in formula I, to form the 2-(dimethylamino)-ethenyl-alkanones of the formula VII

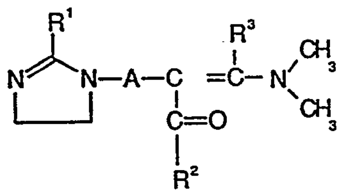

VII

wherein A, $R^1$, $R^2$, $R^3$ have the meanings as defined in formula I, that these compounds of the formula VII are reacted with cyanocetamide under basic conditions to form the 2(1H)-pyridones of the formula I, wherein $R^4$ mans CN, and, if desired, that these nitriles of the formula I are converted into the corresponding acid amides of the formula I

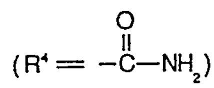

or acids of the formula I ($R^4$ = COOH) by hydrolysis, that these componds are converted by thermic decarboxylation into the corresponding compounds of the formula I, wherein $R^4$ = H, or that the acid amides of the formula I are converted into the corresponding amines of the formula I ($R^4 = NH_2$) by the Hofmann-decomposition.